(19) **Europäisches Patentamt**
**European Patent Office**
**Office européen des brevets**

(11) **EP 3 745 038 A1**

(12) **EUROPEAN PATENT APPLICATION**

(43) Date of publication:
**02.12.2020 Bulletin 2020/49**

(51) Int Cl.:
**F24F 11/30** *(2018.01)*    **F24F 11/63** *(2018.01)*

(21) Application number: **20176358.8**

(22) Date of filing: **25.05.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.05.2019 IT 201900007449**

(71) Applicant: **Generali Jeniot S.p.A.**
**20145 Milano (IT)**

(72) Inventors:
• **VIA, Alessandro**
**20129 MILANO (IT)**
• **SERRA, Michele**
**20137 MILANO (IT)**

(74) Representative: **Modiano, Micaela Nadia et al**
**Modiano & Partners**
**Via Meravigli, 16**
**20123 Milano (IT)**

(54) **DEVICE, SYSTEM AND METHOD FOR MONITORING AIR QUALITY IN A CLOSED ENVIRONMENT**

(57)    A monitoring device (10) for monitoring air quality in a closed environment (A), which comprises: one or more detection devices (21-29), each one configured to detect one or more chemical or physical parameters in the closed environment (A); a data link unit (30), adapted to functionally connect the monitoring device (10) with at least one data communication network (R); and an electronic control and processing unit (40), which is functionally connected to the detection devices (21-29), is configured to activate repeatedly the detection devices (21-29) so as to perform repeated detections of the parameters, and is configured to perform a processing of the parameters detected. The electronic control and processing unit (40) is configured to activate the detection devices (21-29) cyclically and asynchronously, in a sequence that is such as to activate simultaneously only the detection devices (21-29) that have between them a distance that exceeds a preset threshold distance.

Fig.1

**Description**

The present invention relates to a monitoring device for monitoring air quality in a closed environment, which is particularly, although not exclusively, useful and practical for monitoring domestic air quality.

It is known that exposure to pollution indoors (that is to say, in closed environments) in the last fifty years has increased with the migration of the population toward cities and the progressive "tertiarization" of activities: numerous studies of the use of time by various different population groups in the most developed countries have found that people spend very little time outdoors; most of their time is spent at home, in the office, at school, and in vehicles. Therefore the main source of exposure to some atmospheric pollutants is represented by the contamination of indoor air. The problem is even more important for some population groups who are particularly sensitive, such as for example the elderly, children, and people with respiratory diseases.

Indoor pollutants, in particular, are numerous and can originate from various different sources. Their concentration can vary over time and depends on the nature of the source, on ventilation, on habits and on the activities carried out by the occupants in the affected environments. The composition of air indoors is often characterized by a mixture of compounds that can vary greatly with respect to what can be found in the atmospheric air outside.

Therefore, over the years monitoring devices have been developed which are specifically designed to monitor air in closed environments.

These conventional monitoring devices normally comprise one or more sensors which measure the concentration of determined substances in the air, and a data communication system which sends the measurements performed to a data network (typically the internet) in order to make them readable for example by a mobile device like a smartphone.

For example, WO 2011/090763 describes a device for monitoring a plurality of indoor and outdoor environmental parameters and for the wireless transmission of the monitored data to a network, for analysis and distribution online.

Sometimes, conventional monitoring devices for monitoring air in closed environments are provided with acoustic or illuminated indicators which are configured to emit an alarm signal when one of the values detected exceeds a predetermined threshold value.

These conventional monitoring devices for monitoring air in closed environments, although useful and practical, have some drawbacks.

In particular, if there are multiple sensors, these interfere with each other, altering the precision of the detections. In order to overcome this drawback, in the known art, the sensors must be positioned at a certain distance, accordingly increasing the overall space occupation of the monitoring device.

Furthermore, in conventional monitoring devices for monitoring air in closed environments, the number of sensors is normally limited to no more than four, since with a higher number of sensors the complexity and therefore the dimensions and the cost of the device would increase unacceptably.

The aim of the present invention is to overcome the limitations of the known art described above, by devising a monitoring device for monitoring air in closed environments which, for the same encumbrances, is more reliable and precise when compared to the known art.

Within this aim, an object of the present invention is to provide a monitoring device for monitoring air that, for the same encumbrances, makes it possible to monitor a greater number of environmental parameters.

Another object of the invention consists of providing a monitoring device for monitoring air that can monitor the overall air quality in a closed environment in a manner that is particularly simple to interpret.

Another object of the present invention is to provide a monitoring device for monitoring air that is highly reliable, easily and practically implemented, and economically competitive when compared to the known art.

This aim and these and other objects which will become better apparent hereinafter are achieved by a monitoring device for monitoring the air quality in a closed environment, which comprises:

- one or more detection devices, each one configured to detect one or more chemical or physical parameters in the closed environment,
- a data link unit, adapted to functionally connect said monitoring device with at least one data communication network, and
- an electronic control and processing unit, which is functionally connected to said detection devices, is configured to activate repeatedly said detection devices so as to perform repeated detections of said parameters, and is configured to perform a processing of said parameters detected by way of said repeated detections,

characterized in that said electronic control and processing unit is configured to activate said detection devices cyclically and asynchronously, in a sequence that is such as to activate simultaneously only the detection devices that have between them a distance that exceeds a preset threshold distance.

Further characteristics and advantages of the invention will become better apparent from the detailed description of a preferred, but not exclusive, embodiment of the monitoring device for monitoring air quality in a closed environment according to the invention, which is illustrated by way of non-limiting example with the aid of the accompanying drawings wherein:

Figure 1 is a block diagram that schematically illustrates an embodiment of the monitoring device for monitoring air in a closed environment according to the present invention;

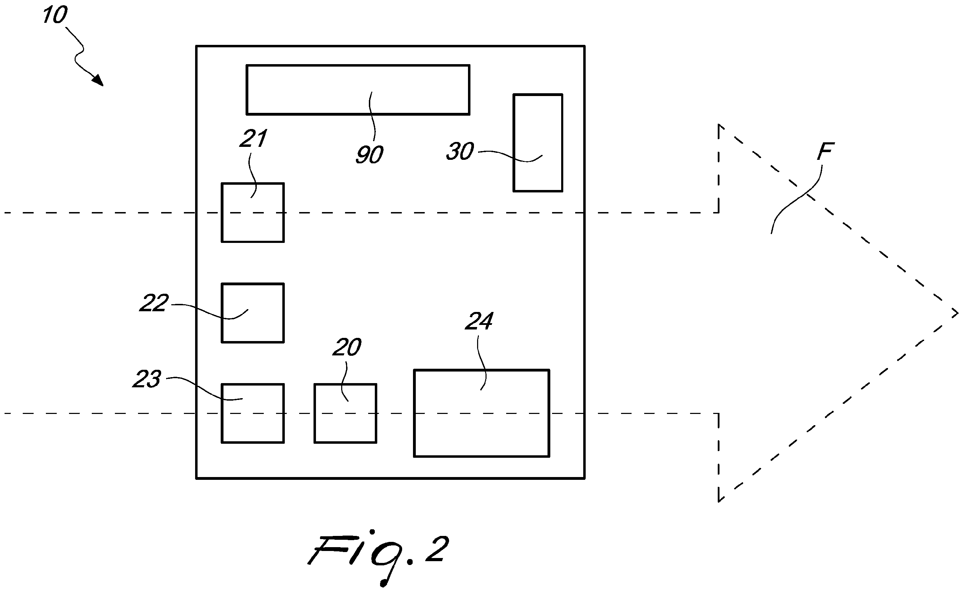

Figure 2 is a schematic diagram of the structure of an embodiment of the monitoring device for monitoring air in a closed environment according to the present invention;

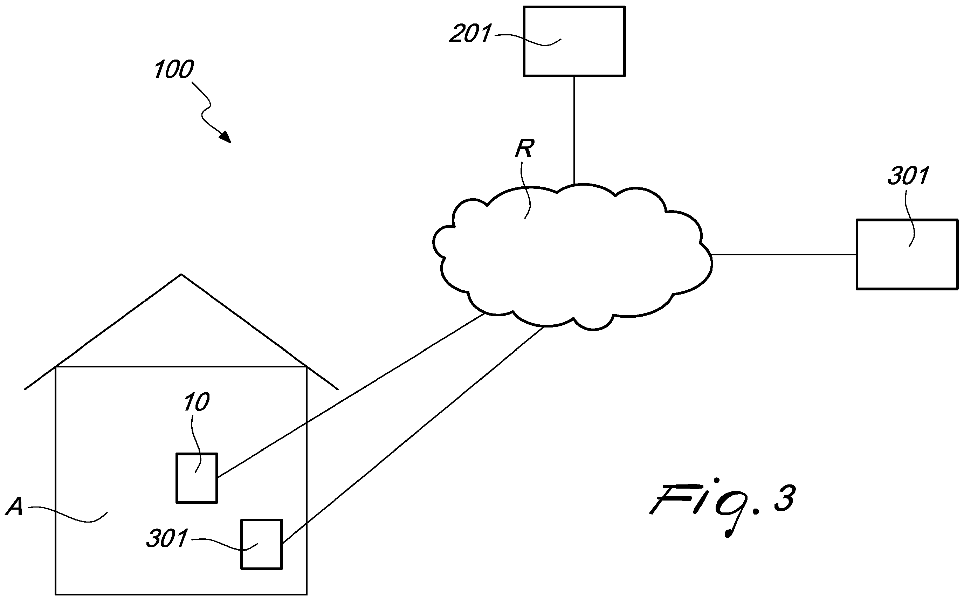

Figure 3 is a block diagram that schematically illustrates an embodiment of the monitoring system for monitoring air in a closed environment according to the present invention;

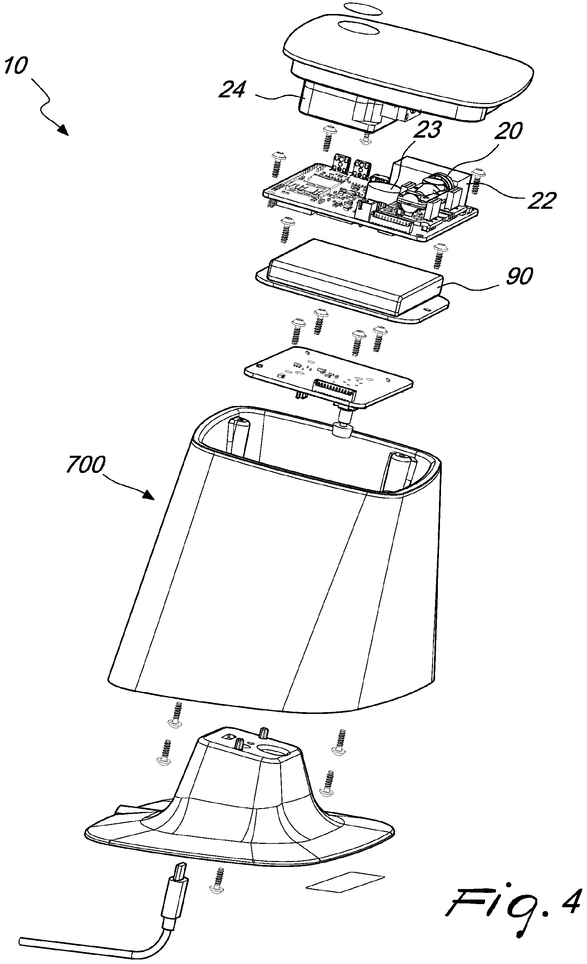

Figure 4 is an exploded view of a second embodiment of the monitoring device for monitoring air in a closed environment according to the present invention;

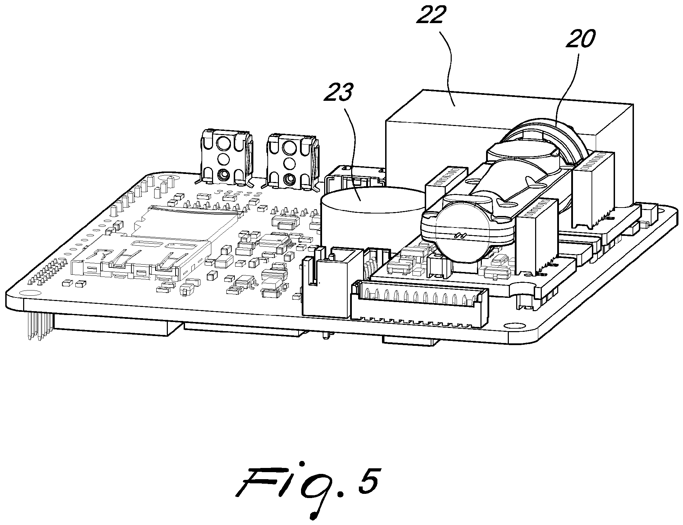

Figure 5 is a detail view of a portion of the embodiment of the monitoring device for monitoring air according to the present invention shown in Figure 4;

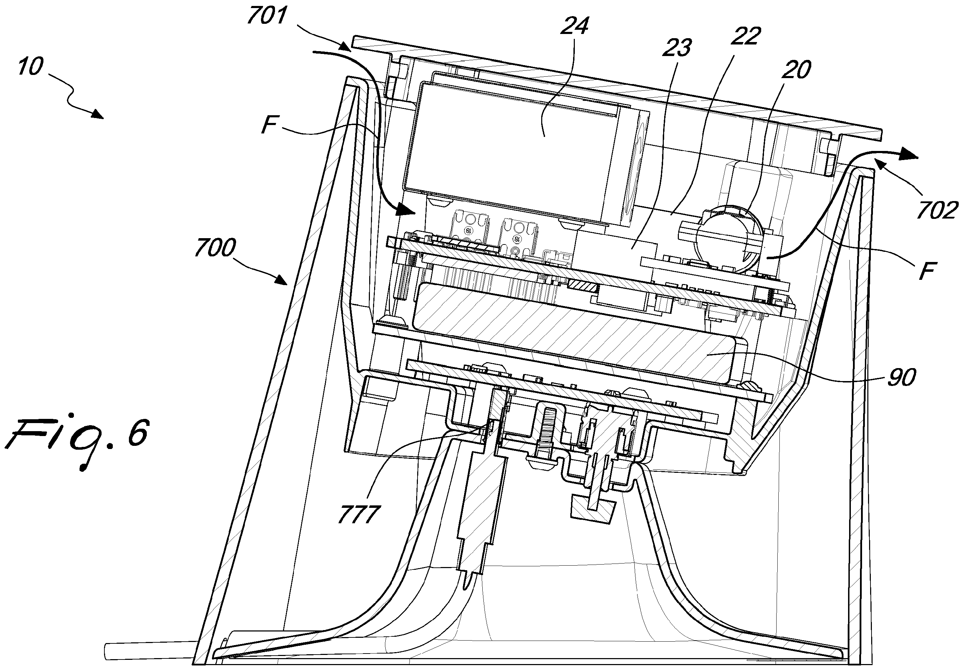

Figure 6 is a cross-sectional view taken along a vertical plane of the embodiment of the monitoring device for monitoring air according to the present invention shown in Figure 4.

With reference to the figures, the monitoring device for monitoring air in a closed environment A is generally designated by the reference numeral 10.

The closed environment A to which reference is made is any indoor environment, such as for example an office, a shop, an apartment, a laboratory etc. In the preferred embodiments, the monitoring device 10 is particularly adapted to monitor air quality in a domestic environment.

It should be made clear from this point onward that the term "monitoring" means the operation of executing measurements, repeated over time, of one or more parameters and optionally processing and/or storing and/or sending these measurements.

The monitoring device 10 therefore comprises one or more detection devices 21-29 each one of which is configured to detect one or more chemical or physical parameters in the environment A and in particular of the air in the environment A, such as for example the concentration of determined gases and/or particles, the temperature, the pressure etc.

Preferably, these detection devices 21-29 comprise a plurality of sensors selected from the group constituted by:

- a $CO_2$ (carbon dioxide) concentration detection sensor 21;
- a $CH_4$ (methane) concentration detection sensor 22;
- a CO (carbon monoxide) concentration detection sensor 23;
- A PM 10/2.5/1 concentration detection sensor 24, i.e. a sensor that is configured to detect the presence and the concentration of PM10 and/or PM2.5 and/or PM1 particulate matter, and is adapted to detect the presence of fine dusts and smoke;

- a humidity sensor 25;
- a brightness sensor 26;
- a temperature sensor 27;
- a pressure sensor 28; and
- an accelerometer 29, adapted to detect the movement and/or the overturning of the monitoring device 10.

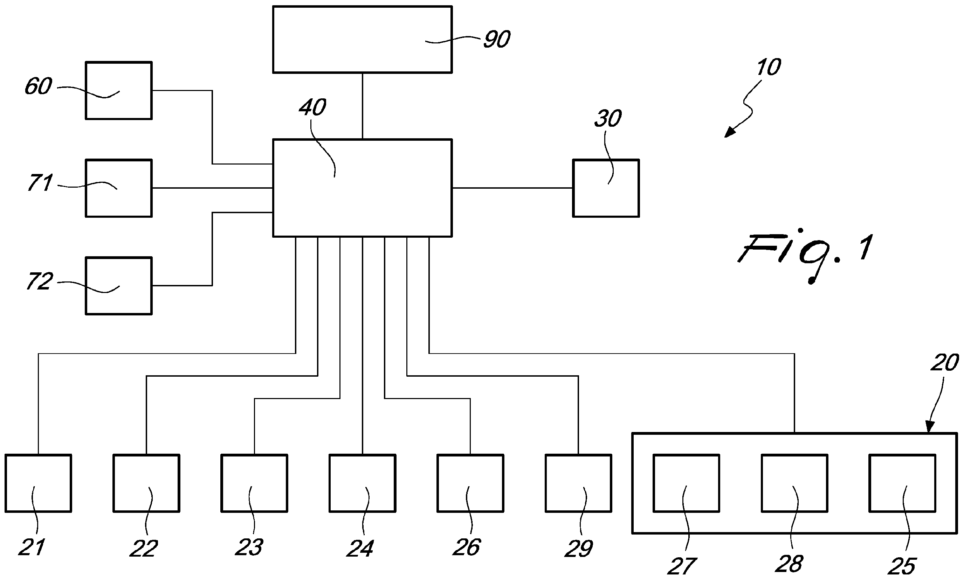

Even more preferably, as shown in Figure 1, all the detection devices 21-29 comprise the sensors just mentioned and therefore at least: a $CO_2$ concentration detection sensor 21, a $CH_4$ concentration detection sensor 22, a CO concentration detection sensor 23, a PM 10/2.5/1 concentration detection sensor 24, a humidity sensor 25, a brightness sensor 26, a temperature sensor 27, a pressure sensor 28, and an accelerometer 29.

In the preferred and illustrated embodiment of Figure 1, the sensors for humidity 25, temperature 27 and pressure 28 are integrated in a single, multifunction detection device 20, such as for example the BME680 sensor made by Bosch® or the like.

The monitoring device 10 according to the invention further comprises at least one data link unit 30, which comprises preferably a radio communication module, and which is adapted to functionally connect, in a known manner, the monitoring device 10 with at least one data communication network, for example to a wireless local network and optionally, through this, to the internet.

Optionally, the data link unit 30 also comprises supplementary radio communication modules, for example short-range and configured according to the Bluetooth or ZigBee standard, for direct connection with other monitoring devices and/or with interface devices that are configured to control and/or read the data sent by the monitoring device 10.

The monitoring device 10 according to the invention comprises an electronic control and processing unit 40 which is functionally connected to the detection devices 21-29, and is configured to activate repeatedly the detection devices 21-29 so as to perform repeated detections of the parameters. The electronic control and processing unit 40 is further configured to performing the processing of the parameters, detected by way of the aforementioned repeated detections.

In other words, the electronic control and processing unit 40 is the programmable central logical unit, which executes mathematical, logical, control, and input/output operations which are programmed, in a known manner, by way of a firmware. The control unit 50 therefore has functions to control the detection devices 21-29 and it handles the data originating from these in order to send them through the data link unit 30 and/or activate one or more reporting and alarm devices 71, 72, which will be described below, as a function of the parameters received.

In the preferred embodiments, the electronic control and processing unit 40 comprises a processor or a microcontroller, suitable memory units and optionally A/D converters.

Conveniently, the electronic control and processing unit 40 is connected with a data storage unit 60, for example comprising a mass storage unit for storing the parameters measured and the processed data. Preferably, the data storage unit 60 comprises a connector which is adapted to connect to a removable mass memory unit, such as for example a flash memory card (an SD card or the like).

Advantageously, the processing of the parameters, which were detected via the aforementioned repeated detections, comprises the calculation of an air quality index Q, by summing the measured parameters with dynamic weightings linked to the deviation of the individual parameter with respect to the mean of all the other parameters that were detected in that same moment. In particular, the air quality index Q is calculated using the following system of formulas:

$$Q(t) = \sum \alpha_i * X_j$$

$$\alpha_i = M \frac{X_N}{(X_i + M X_N)}$$

wherein:

Q(t) = air quality index Q at time t;

$X_j$ = percentage values of the measured parameters with respect to a predetermined threshold value (100% if the value measured is greater than the threshold value);

$\alpha_i$ = dynamic weightings calculated as a function of the N detection devices 21-29 that are activated at that moment, i.e. at time t;

M = mean; and

N(t) = number of detection devices 21-29 that are active at the time of calculation of the value Q(t).

Calculating the air quality index Q makes it possible to have a particularly reliable parameter that is simple to interpret, which indicates the overall quality of the air in the closed environment A.

Note that the calculation considers the number of detection devices 21-29 that are active at time t; in fact, advantageously, the detection devices 21-29 are not all activated simultaneously, but in sequence.

In practice, according to the invention, the electronic control and processing unit 40 is configured to activate the detection devices 21-29 cyclically and asynchronously, in a sequence that is such as to activate simultaneously only the detection devices 21-29 that have between them a distance that exceeds a preset threshold distance. Preferably, this threshold distance is comprised between 10 mm and 14 mm.

In this manner, even if the detection devices 21-29 are positioned very close to each other, the problem of interference between sensors and alteration of the precision of the detections or measurements owing to this interference is avoided.

Conveniently, at least one of the detection devices 21-29 is arranged at a distance that is less than the preset threshold distance from another one of the adjacent detection devices 21-29.

In the preferred embodiments, all or most of the detection devices 21-29 are positioned at a distance that is less than the preset threshold distance from an adjacent detection device 21-29. In this manner it is possible to provide a monitoring device 10 that has a very reduced space occupation.

For example, in an embodiment in which the threshold distance is 12 mm and the detection devices 21-29 comprise all nine sensors described above and they are positioned at a distance of less than 6 mm from each other, the monitoring device 10 can be contained in a cubic box-like body measuring 7 cm on a side.

Conveniently, the activation of the data link unit 30 is done periodically as well, so as to avoid interference with the detection devices 21-29, for example by also being activated by the electronic control and processing unit 40 cyclically and asynchronously with respect to the detection devices 21-29.

Obviously, the monitoring device 10 also comprises a power supply unit 90 which comprises preferably at least one battery, an electric power supply circuit and one or more connectors 777 (for example USB ports) which are adapted to be connected to a mains electricity supply for charging the battery and/or directly powering the monitoring device 10.

Conveniently, the battery and the electric power supply circuit are positioned at a minimum distance of 6 mm from the detection devices 21-29 so as not to generate interference.

According to an optional and advantageous characteristic, the detection devices 21-29 and the electronic control and processing unit 40 are accommodated in a supporting structure 700, in fluid communication with each other, and in such a way that the electronic control and processing unit 40, during operation, generates by convection an air flow F to which the detection devices 21-29 are exposed.

In this manner it is possible to maintain a more compact structure without invalidating the precision of the detections or measurements, in that the detection devices 21-29, even if they are positioned close together and internally, are put in a condition of receiving non-adulterated air that originates directly from outside the monitoring device 10, by virtue of the continuous refresh thereof owing to the flow of air F described above.

In more detail, in a preferred embodiment, the arrangement of the detection devices 21-29 and of the electronic control and processing unit 40 is such that, since the electronic control and processing unit 40, during operation, is subject to a heating comprised between 1°C and 4°C with respect to the ambient temperature, an air flow F is generated that is stable and which has a flow speed comprised between 1 m/sec and 3 m/sec (this should be understood as speed relative to the external air), to which at least some and preferably all of the detection devices 21-29 are directly exposed.

In practice, the supporting structure 700 is provided with an inlet window 701 and an outlet window 702 for the air flow F. The inlet window 701 and outlet window 702 are arranged preferably coaxially and in any case so that the air flow F, in flowing from the former to the latter, affects at least some of the detection devices 21-29.

In order to obtain an effective air flow F as previously described, the inlet window 701 and the outlet window 702 preferably are the same size. Even more preferably, both the windows have the same area (i.e. the same passage section) and therefore the same flow-rate.

In the preferred embodiments, both the inlet window and the outlet window are rectangular in cross-section. In a particular preferred embodiment, which is found to be particularly advantageous from the point of view of compactness and also of the efficiency of the air flow F, both the inlet window and the outlet window have dimensions (width x height) equal to 6 cm x 1.5 cm.

In order to have a sufficient exposure to the air flow F, the detection devices 21-29 are positioned so as to have a mutual distance, in the direction perpendicular to the air flow F, greater than or equal to 0.2 cm.

Also for the same reason, the detection devices 21-29 have a transverse dimension (meaning the width along a dimension transverse to the air flow F) that does not exceed 1 cm.

In addition, inside the supporting structure 700, among the detection devices 21-29 and/or between these and the walls of the supporting structure 700, a passage channel for the air flow F is defined which has a minimum height of at least 0.8 cm and a minimum width of at least 3 cm; that is to say, at the point of lowest height of the passage channel (therefore, for example, at the component whose height closest approaches the upper wall of the supporting structure 700) the height of the passage channel is at least 0.8 cm, and at the point of narrowest width of the passage channel it has a width of at least 3 cm and therefore, ultimately, the channel has a cross-section that at its smallest point is at least 3 cm x 0.8 cm or equal to at least 2.4 cm$^2$.

Turning to the aforementioned reporting and alarm devices 71, 72 in more detail, these comprise preferably at least one light emitter 71 (preferably an LED lamp) and at least one sound emitter 72.

Conveniently, the electronic control and processing unit 40 is configured to activate the reporting and alarm devices 71, 72 as a function of the parameters detected by the detection devices 21-29. For example, in some embodiments, the reporting and alarm devices 71, 72 emit different types of illuminated and/or acoustic signals depending on the physical or chemical parameter that has exceeded the corresponding threshold value. For example, a first acoustic and illuminated signal when the concentration of CO exceeds the corresponding threshold value, a second, different acoustic and illuminated signal when the concentration of $CH_4$ exceeds the corresponding threshold value, and so on.

According to an optional and advantageous characteristic, the electronic control and processing unit 40 is configured to deactivate one or more of the alarm signals when the accelerometer detects the overturning of the monitoring device 10. In this manner, the alarm signal can be deactivated rapidly and at the same time it is impossible to deactivate it by mistake; furthermore it is evident, from the overturned position of the monitoring device 10, that the signal has been deactivated.

In the preferred embodiments, the monitoring device 10 is configured to form part of a monitoring system 100 where the monitoring device 10 is functionally connected, via the data communication network R, to a remote platform 201 (for example a server) which, by way of adapted software, manages the parameters that are detected by the detection devices 21-29 and processed by the electronic control and processing unit 40.

The remote platform 201 is configured to send to one or more interface devices 301, such as for example smartphones or the like, again via the data communication network R, the parameters of the detections and/or information as a function of these parameters (statistics, suggested corrective actions etc.) and/or alarm signals in the event of these parameters exceeding predetermined threshold values.

Operation of the monitoring device 10 for monitoring air quality in a closed environment is clear and evident from the foregoing description.

In practice it has been found that the monitoring device for monitoring air quality in closed environments according to the present invention fully achieves the set aim and objects in that, for the same encumbrances, it is more reliable and precise when compared to the known art.

Another advantage of the monitoring device for monitoring air quality in closed environments according to the invention consists in that, for the same encumbrances, it makes it possible to monitor a greater number of environmental parameters

Another advantage of the monitoring device for monitoring air quality in closed environments according to the invention consists in that it makes it possible to monitor the overall air quality in a closed environment in a manner that is particularly simple to interpret.

Another advantage of the monitoring device of the air quality in closed environments according to the invention consists in that it is highly reliable, easily and practically implemented, and economically competitive when compared to the known art.

The invention, thus conceived, is susceptible of numerous modifications and variations, all of which are within the scope of the appended claims. Moreover, all the details may be substituted by other, technically equivalent elements.

In practice the materials employed, provided they are compatible with the specific use, and the contingent dimensions and shapes, may be any according to requirements and to the state of the art.

The disclosures in Italian Patent Application No. 102019000007449 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1.  A monitoring device (10) for monitoring air quality in a closed environment (A), which comprises:

    - one or more detection devices (21-29), each one configured to detect one or more chemical or physical parameters in the closed environment (A),
    - a data link unit (30), adapted to functionally connect said monitoring device (10) with at least one data communication network (R), and
    - an electronic control and processing unit (40), which is functionally connected to said detection devices (21-29), is configured to activate repeatedly said detection devices (21-29) so as to perform repeated detections of said parameters, and is configured to perform a processing of said parameters detected by way of said repeated detections,

    **characterized in that** said electronic control and processing unit (40) is configured to activate said detection devices (21-29) cyclically and asynchronously, in a sequence that is such as to activate simultaneously only the detection devices (21-29) that have between them a distance that exceeds a preset threshold distance.

2.  The monitoring device (10) according to claim 1, **characterized in that** said preset threshold distance is comprised between 10 mm and 14 mm.

3.  The monitoring device (10) according to claim 2, **characterized in that** at least one of said detection devices (21-29) is arranged at a distance that is less than said preset threshold distance from another one of said adjacent detection devices (21-29).

4.  The monitoring device (10) according to any one of the preceding claims, **characterized in that** said detection devices (21-29) and said electronic control and processing unit (40) are accommodated in a supporting structure (700), in fluid communication with each other, and in such a way that said electronic control and processing unit (40), during operation, generates by convection an air flow (F) to which said detection devices (21-29) are exposed.

5.  The monitoring device (10) according to claim 4, **characterized in that** said air flow (F) is stable and has a flow speed comprised between 1 m/sec and 3 m/sec.

6.  The monitoring device (10) according to any one of the preceding claims, **characterized in that** said detection devices (21-29) comprise a plurality of sensors selected from the group constituted by:

    - a $CO_2$ concentration detection sensor (21);
    - A $CH_4$ concentration detection sensor (22);
    - A CO concentration detection sensor (23);
    - A PM 10/2.5/1 concentration detection sensor (24);
    - a humidity sensor (25);
    - a brightness sensor (26);
    - a temperature sensor (27);
    - a pressure sensor (28); and
    - an accelerometer (29).

7.  The monitoring device (10) according to any one of the preceding claims, **characterized in that** said detection devices (21-29) comprise: a $CO_2$ concentration detection sensor (21), a $CH_4$ concentration detection sensor (22), a CO concentration detection sensor (23), a PM 10/2.5/1 concentration detection sensor (24), a humidity sensor (25), a brightness sensor (26), a temperature sensor (27), a pressure sensor (28), and an accelerometer (29).

8.  The monitoring device (10) according to any one of the preceding claims, **characterized in that** said processing of said parameters comprises the calculation of an air quality index Q, calculated according to the following system of formulas:

$$Q(t) = \sum \alpha_i * X_j$$

$$\alpha_i = M \frac{X_N}{(X_i + M X_N)}$$

wherein:

Q(t) = air quality index Q at time t;

$X_j$ = percentage values of the measured parameters with respect to a predetermined threshold value;

$\alpha_i$ = dynamic weightings calculated as a function of the N detection devices (21-29) that are activated at time t;

M = mean; and

N(t) = number of detection devices (21-29) that are active at the time of calculation of the value Q(t).

9. The monitoring device (10) according to any one of the preceding claims, **characterized in that** it comprises one or more interface and alarm devices (71, 72), which are activated by said electronic control and processing unit (40) as a function of the detections performed by said detection devices (21-29).

10. A monitoring system (10) for monitoring air quality in a closed environment (A), **characterized in that** it comprises a monitoring device (10) according to any one of the preceding claims, said monitoring device (10) being functionally connected, via said data communication network (R), to a remote platform (201) which is configured to manage said parameters which are detected by said detection devices (21-29) and which are processed by said electronic control and processing unit (40);
said remote platform (201) is configured to send to one or more interface devices (301), via said data communication network (R), said parameters which are detected by said detection devices (21-29) and/or information as a function of said parameters and/or alarm signals if predetermined threshold values are exceeded by said parameters.

11. A method for monitoring air quality in a closed environment (A), by means if a device that comprises:

- one or more detection devices (21-29), each one configured to detect one or more chemical or physical parameters in the closed environment (A),
- a data link unit (30), adapted to functionally connect said monitoring device (10) with at least one data communication network (R), and
- an electronic control and processing unit (40), which is functionally connected to said detection devices (21-29), is configured to activate repeatedly said detection devices (21-29) so as to perform repeated detections of said parameters, and is configured to perform a processing of said parameters detected by way of said repeated detections,

**characterized in that** it comprises the step of making said electronic control and processing unit (40) activate said detection devices (21-29) cyclically and asynchronously, in a sequence that is such as to activate simultaneously only the detection devices (21-29) that have between them a distance that exceeds a preset threshold distance.

Fig.1

EP 3 745 038 A1

Fig. 2

Fig.3

*Fig.4*

Fig.5

Fig.6

EP 3 745 038 A1

Europäisches
Patentamt

European
Patent Office

Office européen
des brevets

# EUROPEAN SEARCH REPORT

Application Number

EP 20 17 6358

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2011/090763 A2 (UNIV CALIFORNIA [US]; NIEMEYER GREG [US] ET AL.) 28 July 2011 (2011-07-28) * paragraphs [0010], [0011]; figure 1c * | 1-11 | INV. F24F11/30 F24F11/63 |
| A | US 2016/061476 A1 (SCHULTZ RICHARD DOUGLAS [US] ET AL) 3 March 2016 (2016-03-03) * paragraphs [0028] - [0029]; figure 1 * | 1-11 | |
| A | US 5 801 297 A (MIFSUD JEAN CHRISTOPHE [FR] ET AL) 1 September 1998 (1998-09-01) * column 8, lines 12-24; claims 1-2; figure 1 * | 1-11 | |

TECHNICAL FIELDS
SEARCHED (IPC)

F24F
G01N

The present search report has been drawn up for all claims

| Place of search | Date of completion of the search | Examiner |
|---|---|---|
| Munich | 19 October 2020 | Degen, Marcello |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 17 6358

This annex lists the patent family members relating to the patent documents cited in the above-mentioned European search report.
The members are as contained in the European Patent Office EDP file on
The European Patent Office is in no way liable for these particulars which are merely given for the purpose of information.

19-10-2020

| Patent document cited in search report | | Publication date | Patent family member(s) | | Publication date |
|---|---|---|---|---|---|
| WO 2011090763 | A2 | 28-07-2011 | CN | 102812501 A | 05-12-2012 |
| | | | EP | 2519936 A2 | 07-11-2012 |
| | | | US | 2013038470 A1 | 14-02-2013 |
| | | | WO | 2011090763 A2 | 28-07-2011 |
| US 2016061476 | A1 | 03-03-2016 | NONE | | |
| US 5801297 | A | 01-09-1998 | AT | 177203 T | 15-03-1999 |
| | | | DE | 69416842 T2 | 23-09-1999 |
| | | | DK | 0719411 T3 | 04-10-1999 |
| | | | EP | 0719411 A1 | 03-07-1996 |
| | | | ES | 2131213 T3 | 16-07-1999 |
| | | | FR | 2710153 A1 | 24-03-1995 |
| | | | GR | 3030346 T3 | 30-09-1999 |
| | | | JP | H11500821 A | 19-01-1999 |
| | | | US | 5801297 A | 01-09-1998 |
| | | | US | 5918257 A | 29-06-1999 |
| | | | WO | 9508113 A1 | 23-03-1995 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011090763 A

- IT 102019000007449